# EUROPEAN PATENT APPLICATION

(11) **EP 1 522 322 A1**
(43) Date of publication of application: **13.04.2005**
(21) Application number: 03022780.5
(22) Date of filing: 10.10.2003
(51) Int. Cl.: A61L 27/02, A61L 27/04

(54) **Cartilage regeneration**

(71) Applicant: Witte, Frank, Dr., 30625 Hannover (DE)
(72) Inventor: Witte, Frank, 30625 Hannover (DE); Wirth, Carl-Joachim, 30916 Isernhagen (DE); Windhagen, Henning, 30159 Hannover (DE); Kaese, Volker, 30161 Hannover (DE)
(74) Representative: Kröncke, Rolf, Dr.

(57) **Abstract**

The present invention relates to the use of magnesium or magnesium derivatives for promoting the regeneration and/or growth of cartilaginous tissue. In particular, the present invention relates to the use of magnesium or magnesium derivatives in form of a substrate or an agent for the prophylaxis or treatment of cartilage diseases, disorders or damages. Further, the invention relates to the use of magnesium or magnesium-derivatives in tissue engineering and/or gene therapy.

## Description

The present invention relates to the use of magnesium (Mg) or Mg-derivatives as substrates or agents for the prophylaxis or treatment of cartilages diseases, disorders or damages. In particular, the present invention relates to the ability of Mg ions to stimulate the growth and regeneration of cartilaginous tissue. Especially, Mg is able to regenerate hyaline cartilage, elastic cartilage and/or fibrocartilage.

### Background art

Different types of cartilages are determined by chondrocytes and their extracellular matrix (ECM). These different types of cartilage provide different anatomical and functional properties according to their histological morphology. In particular, chondrocytes, the main cellular component of the cartilage, are slow growing cells that secret extracellular matrix proteins to form the different types of cartilages found in the body. Depending on the structure of the cartilage, the cartilaginous tissue appears histologically as hyaline, elastic or fibrocartilage. A combination of these tissues in one functional unit is possible.

Hyaline cartilage represents the most common type of cartilage in the body and contains characteristally collagen type II fibres in its ECM. Typically, hyaline cartilage can be found in articular joints, costal cartilage (ribs), nose, larynx, and growth plate. Another type of cartilage is the elastic cartilage. This kind of cartilage may be found in ear, trachea and larynx, i.e. the epiglottis. The third type of cartilaginous tissue is present in symphysis, intervertebral disci, parts of the articular joints, menisci and in other joints, like the jaw-joint.

Of course, it is possible to find a combination or intermediates of these types of cartilage. For example, the epiphyseal cartilage in the growth or cartilage plate.

In many diseases and disorders a damage of the cartilage occur. However, regardless of their etiology, cartilage defects, e.g. of articular joints, and their treatment remain one of the unsolved problems in medicine.

For example, in case of cartilage diseases of articular joints, there are two major diseases that affect cartilage, namely osteoarthritis and rheumatoid arthritis; both osteoarthritis and rheumatoid arthritis result in degradation and degeneration of the articular cartilage. Osteoarthritis is primarily a non-inflammatory disorder of movable joints characterized by an imbalance between the synthesis and degradation of the articular cartilage, leading to the classic pathologic changes of wearing away and destruction of cartilage. The major symptoms of osteoarthritis are pain, stiffness, crackling, and enlargement and deformities of the affected joints; at the early stage of osteoarthritis there is little inflammation and swelling of the joints, however in advanced stages swelling and inflammation is usually present. Rheumatoid arthritis is an autoimmune systemic disease accompanied by severe inflammation of the joints. In most patients rheumatoid arthritis begins with a general feeling of malaise, fatigue, often accompanied by diffuse musculoskeletal pain. Eventually the disease progresses resulting in pain on motion, tenderness, swelling and deformation of multiple joints; because rheumatoid arthritis is a systemic disease, it may be accompanied by extra-articular complications, such as anemia, vasculitis, scleritis, pleurisy, pericarditis, and peripheral neuritis.

Further, damage of cartilage and/or the underlying bone occurs post-traumatically or in orthopaedic surgery. In these cases regeneration or replacement of the destroyed cartilaginous tissue is necessary.

Moreover for the re-building of cartilage, e.g. cartilage present in ear, nose, intervertebral disci or menisci, it is necessary to engineer new cartilage in vitro and, subsequently, transplant the in vitro generated cartilaginous tissue to the patient. However, re-building or tissue engineering of cartilage tissue presently requires the use of a scaffold, cells, preferably obtained from the patient to be treated, and a cocktail of various growth and differentiation factors. In particular the necessity of using a cocktail of various growth and differentiation factors renders this method expensive. Moreover, the scaffold is often a material which does not degrade after transplantation but remain in the body.

### Summary of the present invention

In view of the above described problems, the object of the present invention is to improve the growth and regeneration of cartilaginous tissue.

In particular, the invention relates to the use of Mg or Mg-derivatives in form of a substrate or an agent for the prophylaxis or treatment of cartilage diseases, disorders or damages.

Especially, the present invention relates to the use of Mg or Mg-derivatives in the treatment of cartilaginous tissue after orthopaedic surgery or posttraumatic and/or degenerative damage of the cartilage.

The invention describes *inter alia* methods and compositions for the treatment of rheumatoid arthritis and osteoarthritis and other conditions that manifest cartilage degradation and inflammation particularly of the joints, but including other cartilages.

### Short description of the figures

- Figure 1.: 3D reconstructed micro-CT data showing an automatically segmented residual volume of the magnesium cylinder in the medial femur condyle 4 weeks postoperatively.
- Figure 2.: O'Driscoll score values of treatment side and control side at different times postoperatively. Data are expressed as mean ± standard deviation (SD).
- Figure 3.: Histological sections of regenerated tissue in osteochondral defects 4 weeks postoperatively on the control side (L) and on the treatment side (R).
Sections were stained by safranin-O. Bar = 1 mm.

### Detailed description of the invention

It is noted that as used herein the following terms have the meaning as indicated below.

The term "Mg or Mg-derivatives" is intended to mean the salts including complexed forms, free ion form or the pure metal of magnesium (Mg).

The term "substrate" is intended to mean a porous or non-porous matrix structure, e.g. a scaffold, a patch, a foam, a sponge, calciumphosphates, like alpha-, beta- TCP, Hydroxylapatit, mixtures of different calciumphosates, etc; a gel, especially hydrogels; solids, like pure metal, magnesium alloys, other metal alloys containing magnesium; pharmaceutically acceptable carrier, polymers, which may be degradable; or implants. Additionally, the term encompasses fastenings means for bones, like screws, nails, rings, anchor, pins, spacer, cages, cerclages, plates, parts of prosthesis, wires woven or drilled bands; sutures for surgery, like wires, thread, films, or web; clips; clamps; staples; prosthesis; medical means, like instruments and devices for surgery; which may be coated or which may consist of a material comprising Mg or Mg-derivatives. Moreover, abrasive agents for sectioning tissueby means of a jet are encompassed herein.

The term "agent" means a pharmaceutical or medicinal composition containing as an active ingredient Mg or Mg-derivatives as defined herein. In particular. The agent may be in form of a tablet, injection or infusion to be applied locally For example, the Mg is present in solution as a complexed form or as magnesium ions.

The term "cartilaginous tissue" or "cartilage" as used herein means any type of cartilage or tissue comprising cartilage-like structures. In particular, the above terms encompass the hyaline-, elastic-, and fibrocartilage and intermediates or mixed structures thereof.

The magnesium cation is an essential mineral for many animals, including mammals, and especially for humans. As used herein, the term "magnesium". As such, magnesium is also a cofactor in numerous enzymatic reactions. It is involved in phosphate transfer from ADP and ATP muscle contractility, and neuronal transmission. The majority of magnesium in the human body is located in the bones in the form of phosphates and carbonates, and the remainder is found principally in the liver and muscles; red blood cells also contain magnesium. Magnesium inhibits nerve impulses and relaxes muscle contractions, thereby functioning antagonistically to calcium. On the other hand, like calcium, magnesium can bind phosphates and can substitute for calcium as a bone or tooth mineral.

Thus, only about 1% of the total magnesium present in the body is in the extracellular, liquid compartment, mainly in the serum. The magnesium concentration in the serum is typically about 1,8 to 2,2 mg/dl; 0,9 mmol/l.

In the blood serum, magnesium can be found mainly in three different forms, i.e. protein-bound magnesium, complexed magnesium or magnesium ions.

The distribution of magnesium varies with age and within different species. That means e.g. the concentration of magnesium in bones and menisci decrease with age.

Various magnesium compounds have been used via intramuscular, oral, and intravenous routes of administration. For example, magnesium acetate is used as a source of magnesium and as an acetate supply of bicarbonate in hemodialysis or peritoneal dialysis solutions; magnesium chloride is likewise used in dialysis solutions.

The inventors now found that the local application of magnesium enhance the tissue regeneration in cartilaginous tissue. In particular, elevating the local extracellular magnesium concentration above physiologic level leads to enhanced tissue regeneration. In addition, the "quality" of the regenerated tissue was increased. This means, the cartilage surface was smoother and the forming of scar-like tissue was decreased.

The substrate to be used in the claimed method for the prophylaxis or treatment of cartilage damages due to diseases or traumas or surgery is preferably a degradable substrate. For example in case of coatings, the coating comprising magnesium is bio-degradable and, thus, the concentration of magnesium in the extracellular compartment of the cartilaginous tissue is temporarily elevated above physiologic level.

Further, the same effect may be obtained using a porous or non-porous material as indicated above, which degrades after implantation and/or, when used in tissue engineering, in cell culture. Alternatively, magnesium can be administered temporarily by controlled drug-release formulations, infusion, injection or catheter. For example a daily injection may be administered for about four weeks.

Thus, the regulated elevation of magnesium concentration above physiologic level by applying magnesium in form of a substrate or agent accelerates cartilage growth and/or regeneration in mammals.

Preferably, the magnesium concentration is at least three times above the physiologic level, more preferably five times above the physiologic level of the respective compartment, i.e. the extracellular compartment of the cartilage or, when used in tissue engineering, of the cell culture medium.

In another aspect of the invention it is provided a method of using magnesium or magnesium-derivatives as defined herein to promote specific stages of chondrocyte and/or cartilage maturation. Thus, by time-controlled application it is possible to regulate osteogenesis and chondrogenesis of the regenerating bone and cartilaginous tissue in order to optimise the newly formed structure of the cartilage.

In a further embodiment, the substrate or agent containing Mg or Mg derivatives allows for differently regulated release of predetermined amounts of Mg into the environment. Thus, it is possible to promote or suppress specific stages of cartilage development.

In addition, the present invention allows to control the growth and development of artifical cartilage, e.g. for the use as a framework for organs. These artifical organs may be used in the replacement of the outer ear, valvular, nose or intervertebral discs or for the replacement of menisci or articular joints.

In another embodiment, Mg or Mg-derivatives are used in in vitro tissue engineering of cartilaginous tissue. Methods for the generation of cartilaginous tissue via tissue engineering is known in the art. However, the methods presently described requires the use of expensive cocktails of various growth factors or differentiating factors along with the respective chondrocytes or chondroblasts, bone-marrow stromal cells, synovial cells and various precursor cells. The use of Mg or Mg derivatives as defined herein allows for the reduction of other growth factors thus reducing the costs and, additionally, allows for a controlled generation and optimized development of the cartilage.

In addition, Mg and Mg-derivatives can be used in a method for the preparation of cartilaginous tissue in gene therapy.

Also encompassed is the method of treating patients suffering on cartilage diseases, disorders or damages due to surgery, trauma, degeneration or as a consequence of other types of diseases. The Mg or Mg-derivatives may be administered systemically or in a single dose into or in the vicinity of the cartilage to be treated. The administration may be in form of pharmaceutical compositions like infusions, injections or via catheter. Alternatively, a substrate may be used which allows for the release of Mg or Mg derivatives by bio- and/or chemical and/or physical degradation. When treating patients it is necessary to elevate the level of magnesium in the extracellular compartment of the cartilage above physiologic level, preferably at least 300% above said level.

Thus, the present invention relates to a method of treating or preventing cartilage diseases, disorders or damages characterized in administering magnesium into or the vicinity of the cartilage. Further, the present invention relates to a method for growing or regenerating cartilaginous tissue characterized in elevating the magnesium concentration in the cartilaginous tissue above physiologic level. In particular, the method may comprise administering the magnesium in form of a substrate or an agent.

The local administration of Mg or Mg-derivatives as defined herein allows for an optimised regeneration and/or growth of cartilaginous tissue. In particular by timely limited administration, e.g. by using degradable substrate containing Mg or Mg-derivatives, it is possible to accelerate the tissue regeneration in chondral and/or in osteocartilage defects.

Thus, Mg and Mg-derivatives can positively influence cartilage formation in vivo and in vitro.

Of course, it is possible to combine the Mg or Mg-derivatives being present in form of a substrate or agent with at least one further compound known in the art to promote the growth and/or regeneration of cartilaginous tissue or which is used in the prophylaxis or treatment of cartilage diseases, disorders or damages.

The formulation of suitable substrates and agents in form of pharmaceutical composition is known to the skilled person. Moreover, the dosage of the Mg or Mg-derivatives administered may vary depending on the conditions of the individual, age, body weight, etc. The skilled person knows how to provide Mg or Mg-derivatives in an amount elevating the level of Mg above the physiologic level of the extracellular compartiment of the cartilage or of the culture medium in case of tissue engineering.

In one embodiment the level is increased for a pretermined time, e.g. for 6 to 8 weeks after surgery, and afterwards the level of Mg is slowly decreased to a lower level or even to physiologic level.

When using Mg in form of an implant, the implant may consist of metallic magnesium or may be an alloy containing magnesium as the main component (base element) or as a minor component. In this regard, the alloy may contain further metals having a promoting activity on cartilage formation, e.g. calcium or lithium or manganese.

Thus, the present invention may be used for the prophylaxis or treatment of chondral or osteochondral defects or damages. Further, the present invention is useful in treating the rupture or degeneration of meniscus or discus, like slipped discus. Moreover, the present invention relates to the use of magnesium or magnesium derivatives in degenerative, autoimmune or inflammatory diseases or trauma leading to hyaline, elastic and/or fibroelastic cartilage damage. Further, diseases causing growth disorders or growth disorders itself, which may affect directly or indirectly the cartilaginous tissue of the growth plate are encompassed in the present invention.

The method or use according to the present invention is applicable to mammals, i.e. humans and animals.

Alternatively, magnesium or magnesium derivatives as defined herein are useful for the preparation of artificial meniscus or discus to be used in meniscus or discus replacement, respectively. Moreover, the present invention is useful in ligament surgery. Cartilaginous tissue can e.g. be found on the insertion of the ligament. Thus, ligament surgery may encompass the use of magnesium or magnesium derivatives for promoting the generation of cartilaginous tissue being connected with the ligament.

In another embodiment, the magnesium or magnesium derivatives as defined herein may be used as coatings for permanent implants, like permanent prosthesis, in order to promote the forming of cartilaginous tissue at the interface of implant and surrounding tissue.

### Examples

The following Examples illustrates the effects when using Mg or Mg-derivatives in a regulated manner on the regeneration of cartilage. However, the invention is not limited to or by the examples.

### Example 1

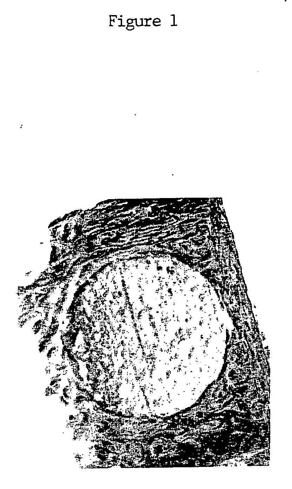

An eight millimeter deep hole (3 mm diameter) was drilled into the medial femur condyle in both knees of fifteen skeletally mature New Zealand White Rabbits (mean weight: 4.0 ± 0.39 kg). Skeletal maturity was verified by analysing the growth plate on postoperatively taken x-rays [Fukuda et al., Exp. Anim. 30: 497-501, 198]. A magnesium cylinder (3 mm diameter, 5 mm height) was insert into the drill hole of the right femoral condyle 3 mm beneath the cartilage surface. The left drill hole was left empty as a control. Animal experiments were conducted under an ethic committee approved protocol in accordance with German federal animal welfare legislation. After the operating procedure, the rabbits were randomly assigned to three time groups of five animals each. The exact depth of the implant beneath the cartilage surface was measured on postoperatively taken radiographs. At 2, 4 and 6 weeks post-operation five animals were euthanized and an osteocartilage slice of 2 mm was cut tangential to the femoral cartilage surface to harvest the regenerative tissue in the osteocartilage defect and the surrounded cartilage. Following decalcification and paraffin embedding, 4 µm thick histological sections were stained by toluidine blue and safranin-O. Histological sections were scored by three blinded investigators using the O'Driscoll score [O'Driscoll et al., Tissue Eng. 7: 313-20, 2001]. To detect implant degradation the remaining femur condyle was scanned by synchrotron-based micro computer-tomography (µCT) at beamline HARWI (HASYLAB at DESY, Hamburg, Germany) at an energy of 31 keV and a resolution of 12.5 µm (Figure 1). The residual implant volume was measured on reconstructed data using visual graphics software. Mann-Whitney test was used to compare data between groups. Correlation between implantation depth and O'Driscoll score value was analyzed by linear regression. Differences with p-values less than 0.05 were considered statistically significant.

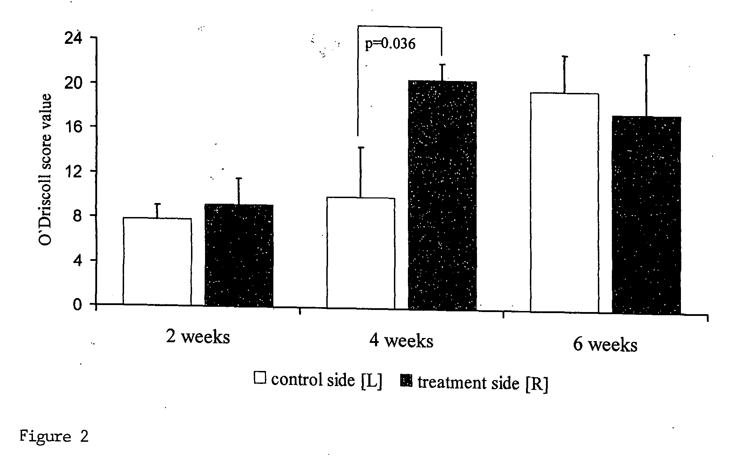

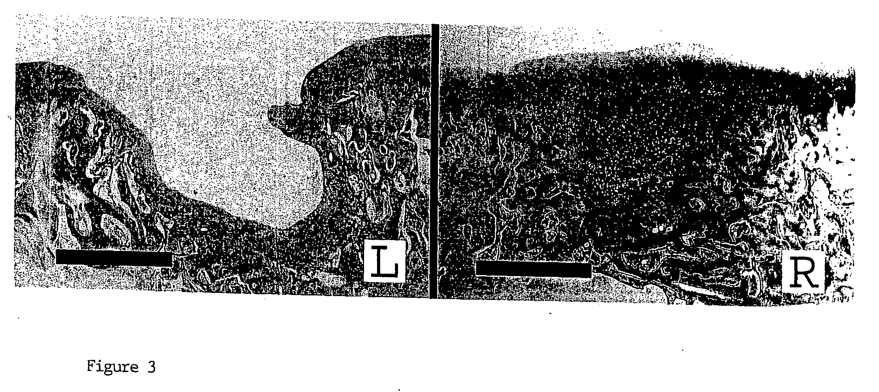

Four weeks postoperatively the regenerative tissue above the degrading magnesium implant had significantly (p=0.036) higher O'Driscoll score values compared to the control side (Figure 2). At four weeks the cartilage surface was smother and the defect filling was more complete in the treatment side than in the contralateral side (Figure 3). The score values at four weeks in the treatment side were as high as the score values at six weeks. At six weeks there was no significant difference observed between the treatment and the control side in histological sections (Figure 2). The average implant depth beneath the cartilage surface was 3.54 ± 0.77 mm. The depth of the implant did not correlated with the O'Driscoll score values of the treatment side (R²=0.177, p=0.232). Micro-CT based volume determinations of the remained magnesium implants revealed continuous implant degradation. Obvious degradation was observed at four weeks (Figure 1).

Thus, the above example shows that degrading magnesium implants have no negative influence on tissue regeneration in osteocartilage defects. In contrast, the regenerative cartilage tissue above the degraded magnesium implant is more mature at four weeks postoperatively compared to the control side (Figure 2). Thus, regenerative cartilage tissue of the knee receiving an Mg-implant displayed accelerated tissue repair in the four week group, the O'Driscoll score values on both sides rose to the same level at six weeks postoperatively. The release of magnesium ions by degradation, which was verified by µCT, enhanced early tissue regeneration in the treatment side. Thus, in this example it is demonstrated that degradable magnesium implants are biocompatible materials suitable for intraarticular use which does not only negatively affect the early stages of cartilage repair but to the contrary promote the regeneration of cartilaginous tissue.

## Claims

1. The use of magnesium (Mg) or Mg-derivatives for the preparation of a substrate or agent for the prophylaxis or treatment of cartilage diseases, disorders or damages.

2. The use of Mg or Mg-derivatives for the preparation of a substrate or agent for promoting the growth and/or regeneration of cartilaginous tissue.

3. The use according to claim 1 or 2 **characterized in that** the cartilage is hyaline cartilage, elastic cartilage and/or fibrocartilage.

4. The use according to any one of the preceding claims wherein the Mg is present as a salt or in a complexed form.

5. The use according to any one of claims 1 to 3 wherein the Mg is present as pure metal or as magnesium ions.

6. The use according to any one of the preceding claims wherein the substrate is a porous matrix or a coating.

7. The use according to any one of the claims 1 to 5 wherein the agent is injection, an infusion and/or a time-release composition.

8. The use according to any one of the preceding claims **characterized in that** the extracellular concentration of the magnesium ion is above its physiologic level.

9. The use according to claim 8 wherein the concentration is at least 3 times above the extracellular physiologic level.

10. The use according to any one of the preceding claims **characterized in that** the magnesium is locally administered.

11. The use according to any one of the preceding claims **characterized in that** the magnesium is temporarily administered.

12. The use according to any one of the preceding claims for the preparation of cartilaginous tissue in vitro.

13. The use according to any one of the preceding claims **characterized in that** the substrate or agent additionally contains at least one compound known to promote the growth and/or regeneration of cartilaginous tissue or used in the prophylaxis or treatment of cartilage diseases, disorders or damages.

14. Method for the preparation of cartilaginous tissue **characterized in** containing the step of adding magnesium to the tissue in concentrations above the extracellular physiologic level of magnesium.

15. Method according to claim 14 for tissue engineering and/or gene therapy of cartilaginous tissue.
